# DEMANDE DE BREVET EUROPEEN

(11) **EP 0 895 998 A1**
(43) Date de publication de la demande: **10.02.1999**
(21) Numéro de dépôt: 98401924.0
(22) Date de dépôt: 28.07.1998
(51) Int. Cl.: C07H 5/06, A61K 7/06

(54) **N-(dialkylamino)alkyl glycosylamines et dérivés, procédé de préparation, et utilisations**

(30) Priorité: 08.08.1997 FR 9710216
(71) Demandeur: CECA S.A., 92800 Puteaux (FR)
(72) Inventeur: Petit, Serge, 74540 Cusy (FR)
(74) Mandataire: Haicour, Philippe

(57) **Abrégé**

L'invention a pour objet des composés de formule : 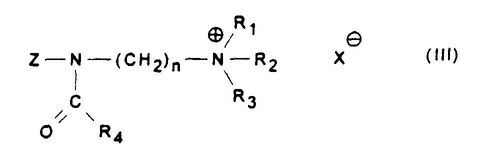 dans laquelle :
Z représente un radical glycosyle,
R₁ et R₂, identiques ou différents, représentent un radical alkyle en C₁-C₄ ou hydroxyalkyle en C₂-C₄,
R₃ représente un atome d'hydrogène ou un radical alkyle en C₁-C₄,
R₄-C = O est un radical acyle aliphatique, linéaire ou ramifié, saturé ou insaturé en C₂-C₂₂,
X représente un atome d'halogène ou un groupe de formule SO₃-O-R₃, R₃ étant nécessairement un radical alkyle tel que défini ci-avant,
n est un nombre entier de 1 à 4.

Elle concerne également les dérivés correspondants obtenus par acylation et quatemisation, ainsi que leur préparation et leurs utilisations.

## Description

La présente invention concerne des N-(dialkylamino)alkyl glycosylamines et les dérives correspondants obtenus par acylation et quaternisation. Elle concerne également leurs procédés de préparation et leurs utilisations.

Les surfactants cationiques en général ont la capacité de s'adsorber sur les surfaces solides, en particulier sur celles qui possèdent une charge globale négative. Ce groupe de surfactants trouve des applications dans de nombreux secteurs de l'industrie chimique, en particulier dans le domaine des cosmétiques. Leurs propriétés sont multiples : dispersion de pigments, agent de floculation ou de précipitation, adjuvant de mouillage ou de broyage, germicide à l'égard des micro-organismes du type Gram positif. En outre, ces surfactants développent des propriétés émulsifiantes quand ils sont associés à des tensioactifs non ioniques, ou à des tensioactifs anioniques lorsqu'ils sont sous la forme de "sels neutres".

Dans le domaine cosmétique et capillaire, la principale fonction des surfactants cationiques est le conditionnement de la peau et des cheveux. Ils pénètrent la chevelure humide et agissent au niveau des interactions entre les cheveux, réduisant ainsi le problème du démêlage au moment du séchage et du peignage. Par ailleurs, ils améliorent la texture et la douceur du cheveu, réduisent les effets dus à l'électricité statique en neutralisant les charges anioniques apparentes, évitent la formation des fourches, contribuent à un meilleur état du cheveu et préviennent les dommages résultant des agressions externes. Enfin, il permettent d'assurer la compatibilité du cheveu avec les autres constituants présents dans les compositions tels que les alcools gras, les esters d'acides gras et les parfums.

Les surfactants cationiques ont cependant l'inconvénient d'être irritants pour la peau et les yeux, voire même de provoquer des dommages irréversibles. Il est nécessaire de les employer avec précaution en tenant compte à la fois de leur nature chimique et de la concentration à laquelle ils sont présents dans les compositions cosmétiques ou capillaires.

Le besoin est donc grand de disposer de nouveaux tensioactifs cationiques susceptibles de présenter un faible pouvoir irritant.

Il a maintenant été trouvé de nouveaux tensioactifs cationiques.

Les tensioactifs selon l'invention répondent à la formule : 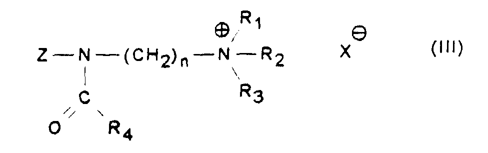 dans laquelle :
Z représente un radical glycosyle,
R₁ et R₂, identiques ou différents, représentent un radical alkyle en C₁-C₄ ou hydroxyalkyle en C₂-C₄,
R₃ représente un atome d'hydrogène ou un radical alkyle en C₁-C₄,
R₄-C = O est un radical acyle aliphatique, linéaire ou ramifié, saturé ou insaturé en C₂-C₂₂,
X représente un atome d'halogène ou un groupe de formule SO₃-O-R₃, R₃ étant nécessairement un radical alkyle tel que défini ci-avant,
n est un nombre entier de 1 à 4.

Un autre objet de l'invention concerne les intermédiaires de synthèse des composés de formule (III) précités, lesquels composés sont définis par les formules : 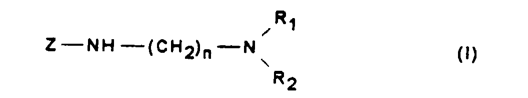 et 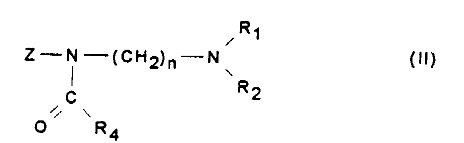 dans lesquelles Z, R₁, R₂, R₄ et n ont la signification donnée précédemment.

Les composés préférés selon l'invention sont les composés de formules (I), (II) et (III) dans lesquelles :
Z représente un résidu de monosaccharide ou d'oligosaccharide réducteur,
R₁ et R₂, identiques ou différents, représentent un radical méthyle, éthyle ou propyle, R₁ et R₂ étant préférentiellement identiques,
R₃ représente un atome d'hydrogène ou un radical méthyle, éthyle ou propyle,
R₄-C = O est un radical acyle de chaîne grasse en C₇-C₁₈,
X représente CI, Br, I ou SO₃-O-R₃, R₃ étant nécessairement un radical alkyle tel que défini ci-avant,
n est égal à 3.

A titre d'exemple de résidus de monosaccharide, on peut citer les résidus de pentoses tels que l'arabinose, le ribose et le xylose, et les résidus d'hexoses tels que le glucose, le galactose, le mannose et le talose. De manière avantageuse, le résidu est choisi parmi les hexoses, et plus particulièrement du glucose (radical glucosyle).

Parmi les résidus d'oligosaccharide, c'est-à-dire des résidus contenant 2 à 20 motifs de monosaccharides, on peut citer les résidus de disaccharides tels que le lactose et le maltose, ou de trisaccharides tels que le maltriose. De manière avantageuse, le résidu est un résidu de disaccharide, et plus particulièrement de lactose (radical lactosyle) ou de maltose (radical maltosyle).

Des exemples de radicaux R₄-C = O sont les restes acyles des acides caprylique, caprique, undécylénique, laurique, palmitique, stéarique ou oléique. Ces acides peuvent par exemple être obtenus à partir d'huiles, d'huiles de coco, d'huiles de suif non hydrogéné ou de suif partiellement ou totalement hydrogéné, et de composés comparables.

Le résidu d'acide gras est souvent un mélange d'au moins deux groupes acyles, par exemple des coupes C₁₂/C₁₄, C₁₆/C₁₈ ou C₁₂ à C₁₈·

L'invention a également pour objet les procédés de préparation des composés de formule (I), (Il) et (III). Ceux-ci seront mieux compris à la lumière de la description ci-après.

### A- Préparation des composés de formule (III)

Le procédé de préparation consiste à faire réagir le composé de formule : 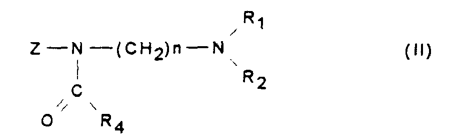
- avec un agent de quaternisation de formule :

   R₃―X (IV)

   dans lesquelles Z, R₁, R₂, R₃, R₄ et n ont la signification donnée précédemment,

L'agent de quaternisation de formule (IV) est choisi parmi les acides halohydriques, les halogénures d'alkyle et les dialkyl sulfates.

La réaction est effectuée en mettant en contact le composé de formule (Il) avec l'agent de quaternisation dans un rapport molaire de 1:0,95 à 1:3, de préférence 1:1 à 1:2.

Bien que la réaction puisse être effectuée en l'absence de solvant, on préfère utiliser un solvant, de préférence anhydre, choisi parmi les alcools tels que le méthanol, l'éthanol, le propanol, l'isopropanol, le butanol ou l'isobutanol, les éther-oxydes tels que le tétrahydrofurane ou l'éther diméthylique de l'éthylène glycol, et les hydrocarbures halogénés tels que le dichlorométhane, le dichloroéthane ou le chloroforme. On préfère les hydrocarbures halogénés.

La quantité de solvant est choisi de telle sorte que, au moment de la mise en contact des réactifs, le composé de formule (II) se trouve à une concentration variant de 3 à 99,9 % en masse, de préférence 5 à 30 %, calculée sur la masse de la solution ou de la dispersion.

La réaction est généralement conduite à une température comprise entre 0 et 130°C, de préférence 30 à 80°C, et à la pression atmosphérique jusqu'à la consommation complète des réactifs ou, le cas échéant, du réactif en défaut.

Le milieu réactionnel contenant le composé de formule (III) peut, le cas échéant, être traité avec de l'eau afin d'hydrolyser l'excès d'agent de quatemisation. Le milieu réactionnel peut être concentré sous une pression comprise entre 0,013 et 101,325 kPa, de préférence 0,013 et 4 kPa. Le résidu ainsi obtenu peut être repris avec de l'eau permutée et lyophilisé, ce qui a pour effet d'améliorer l'aspect du solide résultant.

### B- Préparation des composés de formule (II)

Le procédé de préparation consiste à faire réagir le composé de formule : 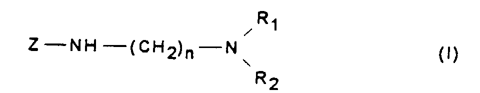
- avec un agent acylant de formule :

   R₄―CO―R₅ (V)
dans lesquelles :
Z, R₁, R₂, R₄ et n ont la signification donnée précédemment et,
R₅ représente un atome d'halogène, de préférence CI, un groupe O-CO-R₆ dans lequel R₆ est une chaîne hydrocarbonée linéaire ou ramifiée, saturée ou insaturée en C₁-C₂₁, de préférence C₆-C₁₇, ou un groupe O-R₇ dans lequel R₇ est un radical alkyle en C₁-C₄ ou diglycéryle dont les résidus acyles, identiques ou différents, renferment 2 à 22 atomes de carbone, de préférence 7 à 18 atomes.

Lorsque l'agent acylant est un anhydride d'acide, on préfère qu'il soit symétrique (R₄ = R₆).

La réaction d'acylation est généralement effectuée en mettant en contact le composé de formule (IV) avec l'agent acylant dans un rapport molaire de 1:0,95 à 1:4, de préférence 1:1 à 1:3, en l'absence de solvant ou en présence d'un solvant tel que l'eau, un alcool à chaîne courte par exemple le méthanol, l'éthanol ou l'isopropanol, ou un mélange hydroalcoolique d'un ou plusieurs de ces alcools. On préfère utiliser un mélange hydroalcoolique.

Lorsque l'agent acylant est un anhydride ou un halogénure d'acide, la réaction et en général menée à une température comprise entre -10 et +60°C, de préférence -5 et +30°C, et à la pression atmosphérique. Lorsque l'agent acylant est un ester, on opère généralement entre 60 et 150°C, de préférence 70 et 130°C, et à une pression pouvant varier de 0,013 à 101,325 kPa, de préférence 0,013 à 10 kPa.

Le pH du milieu réactionnel est généralement maintenu entre 7 et 11, de préférence 7 et 10, ceci pendant toute la durée de la réaction. On peut pour cela utiliser une base choisie parmi les hydroxydes de formule M(OH)ₓ dans laquelle M représente un métal alcalin ou alcalino-terreux et x est la valence du métal, ou un précurseur d'une telle base, par exemple les carbonates ou les hydrogénocarbonates, notamment d'un métal alcalin tel que le sodium ou le potassium, et les alcanoates de métal alcalin tels que le méthanoate ou l'éthanoate de sodium.

A l'issue de la réaction, c'est-à-dire après consommation complète des réactifs ou, le cas échéant, du réactif en défaut, on récupère le milieu réactionnel contenant le composé de formule (II) qui peut être utilisé tel quel ou subir une étape de purification, par exemple par chromatographie sur de la silice.

### C- Préparation des composés de formule (I)

Le procédé de préparation consiste à faire réagir un monosaccharide ou un oligosaccharide réducteur de formule Z-OH,
- avec une amine de formule : 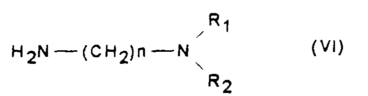 dans lesquelles Z, R₁, R₂ et n ont la signification donnée précédemment, le rapport molaire du saccharide à l'amine étant compris entre 1:0,90 et 1:1,5, de préférence 1:0,95 et 1:1,2.

La réaction peut être conduite indifféremment en l'absence ou en présence d'un solvant tel que l'eau, un alcool ou un mélange d'alcools à chaîne courte, par exemple le méthanol, l'éthanol et l'isopropanol, ou un mélange hydroalcoolique d'un ou plusieurs de ces alcools. On préfère utiliser un alcool lorsque le saccharide est un monosaccharide et un mélange hydroalcoolique lorsqu'il s'agit d'un oligosaccharide.

La réaction est généralement conduite à une température comprise entre 10 et 80°C, de préférence 20 à 70°C, à la pression atmosphérique jusqu'à la consommation complète des réactifs ou du réactif en défaut.

A l'issue de la réaction, le milieu réactionnel peut, le cas échéant, être concentré à une température de 10 à 30°C, de préférence 20 à 25°C, sous un vide de 0,013 à 101,325 kPa, de préférence 0,013 à 4 kPa.

Les composés de formule (II) ou (III) peuvent être utilisés pour préparer des compositions ayant des propriétés tensioactives, notamment des compositions cosmétiques et capillaires.

Les compositions selon l'invention comprennent une base détergente constituée pour tout ou partie d'un ou plusieurs composés de formule (Il) ou (III), d'un véhicule et, le cas échéant, d'additifs.

La base détergente représente généralement 0,1 à 50 % en poids, de préférence 1 à 30 %, de la composition. Elle peut être constituée d'un ou plusieurs composés de formule (Il) et/ou (III), ou d'un mélange d'un ou plusieurs composés de formule (II) et/ou (III) et d'un ou plusieurs tensioactifs connus de l'homme du métier tels que les tensioactifs anioniques, cationiques, non ioniques et amphotères. Les composés selon l'invention représentent généralement 1 à 100 % en poids de la base détergente, et de préférence 10 à 50 %.

Le véhicule est généralement l'eau ou un solvant organique compatible avec une application topique. A titre d'exemple d'un tel solvant, on peut citer l'acétone, l'alcool isopropylique, les triglycérides d'acides gras en C₆-C₂₄, les éthers de glycol tels que les éthers d'alkyle en C₂-C₄ de monoalkylène ou dialkylène glycol, les esters de polyalkylène glycol et d'acide en C₂-C₄, et les silicones volatils.

Les compositions selon l'invention peuvent contenir, en outre, des additifs tels que des corps gras (par exemple des huiles naturelles ou synthétiques), des épaississants ou des gélifiants, des matières actives (par exemple des agents hydratants), des antioxydants, des conservateurs, des parfums et des colorants.

Les compositions selon l'invention peuvent se présenter sous différentes formes. Par exemple, elles peuvent avoir l'aspect d'une lotion moussante ou non moussante, d'une émulsion de consistance liquide ou semi-liquide telle qu'un lait obtenu par dispersion d'une phase grasse dans une phase aqueuse ou inversement, d'une suspension ou d'une émulsion de consistance molle telle qu'une crème ou une pommade, d'un gel ou encore d'une préparation solide telle qu'un "stick", un pain de nettoyage, un tampon imprégné ou un masque hydratant.

Les compositions cosmétiques peuvent être un bain moussant, un gel douche ou un savon liquide doux.

Les compositions capillaires peuvent être un shampooing, par exemple un shampooing doux à usage fréquent, ou un après-shampooing ayant notamment la propriété de conditionner le cheveu.

Les composés selon l'invention peuvent également être employés comme adjuvants de mouillage ou de broyage, agents de floculation ou de précipitation, ou encore comme dispersants de pigments.

Les exemples qui suivent permettent d'illustrer l'invention. Dans ces exemples, on utilise les méthodes d'analyse ci-après :
- Chromatographie sur couche mince (CCM)
   Elle est mise en oeuvre avec des plaques de silice de 200 µm d'épaisseur (taille des particules: 5-10 µm). Le solvant de migration est un mélange n-butanol/acide acétique/eau 1/1/2 (v/v/v) (exemples 1 et 2) ou méthanol/ammoniaque à 25 % 20/1 (v/v) (exemples 3 à 6). Les spots de migration sont révélés par pulvérisation d'une solution de ninhydrine à 0,2 % dans l'acétone et chauffage à 100°C pendant 2 minutes (exemple 1), d'une solution aqueuse d'acide sulfurique à 50 % en volume et chauffage (exemples 2 à 5).
- Résonance magnétique nucléaire (RMN¹³C)
   Les spectres sont réalisés à 50 MHz dans CD₃OD (exemple 1) ou DMSO-d₆ (exemples 2 à 5), ou à 75 MHz dans DMSO-d₆ (exemples 6 à 8). La référence interne est le tétraméthylsilane. Les déplacements chimiques sont exprimés en ppm.
- Spectroscopie infrarouge *(IR)*
   Les spectres sont réalisés sur une pastille de KBr (1 %) ou sur film. Les valeurs sont exprimées en nombre d'onde par cm.

### EXEMPLE 1

### Préparation du composé de formule :

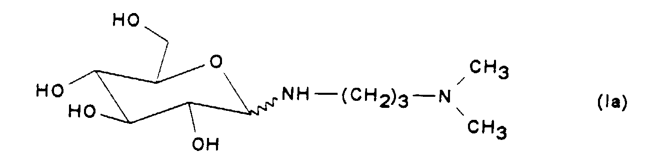

Dans un ballon tricol surmonté d'un réfrigérant muni d'une garde (CaCl₂) et équipé d'un thermomètre et d'une agitation magnétique, on introduit 100 g (0,555 mole) de D-glucose (Réf. 25,307-3; ALDRICH), 57 g (0,557 mole) de 3-diméthylamino propylamine (Réf. D14,500-9 ; ALDRICH) et 660 ml de méthanol (Réf. 17,933-7 ; ALDRICH).

Le milieu réactionnel est agité et maintenu à 40°C pendant 4 heures.

Après refroidissement (environ 20°C), le solvant est évaporé sous pression (2,4 kPa) et on récupère le composé de formule (la) sous la forme d'une huile très visqueuse. Ce composé présente les caractéristiques suivantes :
R_{F} : 0,29
RMN¹³C : 91,87 ; 78,95 ; 74,86 ; 71,85 ; 62,98 ; 45,16 ; 28,79 ; 58,39 ; 45,47.
IR : 3339 ; 2945 ; 2827 ; 1652 ; 1466 ; 1385 ; 1260 ; 1179 ; 1084 ; 1031 ; 897 ; 836.

### EXEMPLE 2

### Préparation du composé de formule :

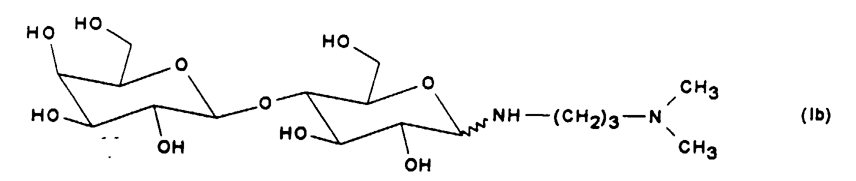

Dans un ballon tricol surmonté d'un réfrigérant et équipé d'un thermomètre et d'une agitation magnétique, on introduit 18 g (0,05 mole) de lactose monohydrate (Réf. L 25-4 ; ALDRICH) solubilisé dans 60 ml d'eau permutée, et 5,1 g (0,05 mole) de 3-diméthylamino propylamine (Réf. D14,500-9 ; ALDRICH) solubilisés dans 60 ml de 2-propanol (Réf. 10,982-7; ALDRICH). Le milieu réactionnel est agité et maintenu à 50°C pendant 6 heures.

Après refroidissement (environ 20°C), le solvant est évaporé sous une pression de 2,2 kPa et le résidu visqueux obtenu est repris avec de l'eau permutée. Après lyophilisation, on récupère le composé de formule (lb) sous la forme d'un solide blanc.

Ce composé présente les caractéristiques suivantes :
R_{F} : 0,52
RMN¹³C : 103,75 ; 90,56 ; 81,29 ; 75,53 ; 75,38 ; 73,14-73,04 ; 70,48 ; 68,04 ; 60,29 ; 57,05 ; 43,71; 27,77.
IR : 3400 ; 2930 ; 2873 ; 1654 ; 1466 ; 1375 ; 1262 ; 1118 ; 1080 ; 1038 ; 780.

### EXEMPLES 3 A 5

### Préparation du composé de formule :

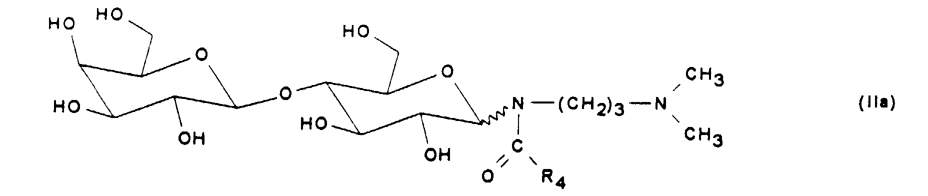

Dans un ballon tricol de 250 ml surmonté d'un réfrigérant et équipé d'un thermomètre, d'une ampoule de coulée et d'une agitation magnétique, on introduit 4,27 g (0,01 mole) du composé (llb) de l'exemple 2, 86 ml d'un mélange eau/éthanol 1/2 (v/v) et 5,3 g (0,05 mole) de carbonate de sodium (Réf. 22,232-1 ; ALDRICH).

On refroidit le milieu réactionnel à 0°C et on y ajoute, en une heure, 0,02 mole de chlorure de l'acide suivant :
- acide undécylénique (4,16 g ; Réf. 16,166-7 ; ALDRICH) : exemple 3
- acide dodécanoïque (4,37 g ; Réf. 15,693-0 ; ALDRICH) : exemple 4
- acide cis-9-octadécénoïque (6,02 g ;Réf. 36,785-0 ; ALDRICH) : exemple 5

Après une heure de réaction, on porte la température du milieu réactionnel à environ 20°C et on filtre (verre fritté n°3). On récupère le filtrat et on le soumet à une évaporation sous vide (20°C ; 2,2 kPa). Le résidu obtenu est purifié sur une colonne de silice (éluant : méthanol/ammoniaque à 25 % 20/1 (v/v)).

Les caractéristiques des produits de formule (lla) ainsi obtenus sont rassemblées dans le tableau suivant.

**TABLEAU 1**

| | | | |
|---|---|---|---|
| EXEMPLE | 3 | 4 | 5 |
| Rendement (%) | 38 | 40 | 33 |
| R_{F} | 0,21 | 0,20 | 0,18 |
| RMN¹³C | | | |
| - partie sucre | 73,21 ; 75,47 ; 76,75 ; 80,01 ; 85,74 ; 103,70 | 60,40 ; 68,14 ; 70,14 ; 70,57 ; 73,26 ; 75,54 ; 76,80 ; 80,15 ; 85,91 ; 103,78 | 60,30 ; 68,05 ; 70,06 ; 70,49 ; 73,14 ; 75,43 ; 76,71 ; 81,26 ; 85,90 |
| | | | |
| - carbonyle | 173,46 | 173,99 | 173,27 |
| | | | |
| - autres | 114,57 ; 138,74 ; 56,15 ; 44,11 ; 32,64 ; 28,81 ; 28,74 ; 28,67 ; 28,43 ; 28,21; 25,54 ; 24,72 | 56,82 ; 45,17 ; 45,10 ; 32,74 ; 31,31 ; 29,05 ; 28,95 ; 28,73 ; 26,39 ; 24,83 ; 22,11; 13,96 | 129,55 ; 56,75 ; 44,95 ; 32,63 ; 31,19 ; 28,74 ; 28,60 ; 28,50 ; 26,49 ; 26,32 ; 24,73 ; 22,01 |
| IR | 3456, 2926, 2855, 1739, 1641, 1557, 1456, 1417, 1181, 1034, 994, 910 | 3398, 2925, 2854, 1646, 1467, 1377, 1120, 1079, 894, 785, 705 | 2921, 2856, 2676, 1737, 1709, 1466, 1375, 1245, 1181, 1036, 723 |

### EXEMPLES 6 A 8

### Préparation du composé de formule :

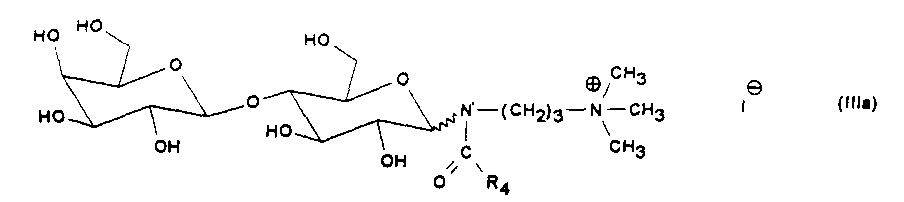

Dans un ballon tricol de 250 ml surmonté d'un réfrigérant muni d'une garde (CaCl₂) et équipé d'un thermomètre et d'une agitation magnétique, on introduit, à 20°C, 5 mmoles du composé (lla) selon l'exemple 3 (2,97 g ; exemple 6), l'exemple 4 (3,1 g, exemple 7) ou l'exemple 5 (3,46 g ; exemple 8), 20 ml de dichlorométhane anhydre et, goutte à goutte, 8 mmoles d'iodométhane (Réf. 1-850-7 ; ALDRICH).

Après 5 heures de réaction au reflux du solvant, on refroidit le milieu réactionnel à environ 20°C et on y ajoute, goutte à goutte, 20 ml d'eau.

Après évaporation du solvant sous pression réduite (20°C ; 2,4 kPa), le résidu est repris avec 20 ml de méthanol et concentré à nouveau dans les conditions précitées. L'opération est renouvelée 3 fois et on récupère le composé de formule (llla).

Les caractéristiques de ces produits sont rassemblées dans le tableau 2 suivant.

**TABLEAU 2**

| | | | |
|---|---|---|---|
| EXEMPLE | 6 | 7 | 8 |
| Rendement (%) | 96,2 | 85,25 | 98,4 |
| RMN¹³C | | | |
| - partie sucre | 60.19 ; 60,25 ; 68,00 ; 69,78; 70,39 ; 73,12 ; 75,31; 75,46 ; 76,71 ; 79,91 ; 85,38 ; 103,67 | 60,36 ; 68,11 ; 69,88 ; 70,50 ; 73,23 ; 75,42 ; 75,57 ; 76,81; 79,96 ; 85,49 | 60,37 ; 68,12 ; 69,88 ; 70,50 ; 73,22 ; 75,46 ; 75,57 ; 76,82 ; 79,95 ; 85,50 |
| | | | |
| - carbonyle | 173,77 | 173,87 | 173,83 |
| | | | |
| - autres | 138,71 ; 114,57 ; 63,49 ; 52,10 ; 37,17 ; 33,08 ; 32,58 ; 28,79 ; 28,73 ; 28,66 ; 28,41 ; 28,18 ; 24,63 ; 22,36 | 63,61 ; 52,21 ; 32,67 ; 31,29 ; 29,03 ; 28,95 ; 28,79 ; 28,72 ; 24,74 ; 22,47 ; 22,10 ; 13,96 | 129,62 ; 63,59 ; 52,21 ; 32,67 ; 31,26 ; 29,17 ; 29,06 ; 28,82 ; 28,67 ; 28,57 ; 26,65 ; 28,56 ; 24,73 ; 22,46 ; 13,95 |

## Revendications

1. Composés de formule : 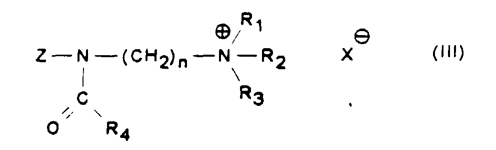 dans laquelle :
Z représente un radical glycosyle,
R₁ et R₂, identiques ou différents, représentent un radical alkyle en C₁-C₄ ou hydroxyalkyle en C₂-C₄,
R₃ représente un atome d'hydrogène ou un radical alkyle en C₁-C₄,
R₄-C = O est un radical acyle aliphatique, linéaire ou ramifié, saturé ou insaturé en C₂-C₂₂,
X représente un atome d'halogène ou un groupe de formule SO₃-O-R₃, R₃ étant nécessairement un radical alkyle tel que défini ci-avant,
n est un nombre entier de 1 à 4.

2. Composés de formule : 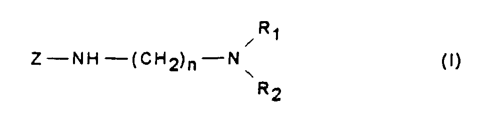 dans laquelle :
Z représente un radical glycosyle,
R₁ et R₂, identiques ou différents, représentent un radical alkyle en C₁-C₄ ou hydroxyalkyle en C₂-C₄,
n est un nombre entier de 1 à 4.

3. Composés de formule : 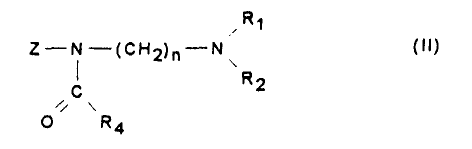 dans laquelle :
Z représente un radical glycosyle,
R₁ et R₂, identiques ou différents, représentent un radical alkyle en C₁-C₄ ou hydroxyalkyle en C₂-C₄,
R₄-C = O est un radical acyle aliphatique, linéaire ou ramifié, saturé ou insaturé en C₂-C₂₂,
n est un nombre entier de 1 à 4.

4. Composés selon l'une des revendications 1, 2 ou 3, pour lesquels :
Z représente un résidu de monosaccharide ou d'oligosaccharide réducteur,
R₁ et R₂, identiques ou différents, représentent un radical méthyle, éthyle ou propyle,
R₃ représente un atome d'hydrogène ou un radical méthyle, éthyle ou propyle,
R₄-C = O est un radical acyle de chaîne grasse en C₇-C₁₈,
X représente CI, Br, I ou SO₃-O-R₃, R₃ étant nécessairement un radical alkyle tel que défini ci-avant,
n est égal à 3.

5. Composés selon la revendication 4, pour lesquels R₁ et R₂ sont identiques.

6. Composés selon l'une des revendications 4 ou 5, pour lesquels Z représente un radical glucosyle, lactosyle ou maltosyle.

7. Procédé de préparation des composés selon la revendication 1, qui consiste à faire réagir le composé de formule : 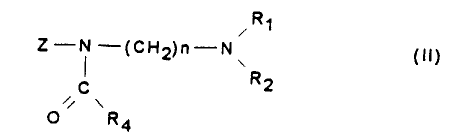
- avec un agent de quaternisation de formule :
R₃―X (IV)
dans lesquelles Z, R₁, R₂, R₃, R₄ et n ont la signification donnée précédemment,

8. Procédé selon la revendication 8, dans lequel le rapport molaire du composé de formule (Il) à l'agent de quaternisation est compris entre 1:0,95 et 1:2.

9. Procédé selon l'une des revendications 7 ou 8, dans lequel la réaction est effectuée dans un solvant.

10. Procédé selon la revendication 9, dans lequel le solvant est choisi parmi les alcools. les éther-oxydes et les hydrocarbures halogénés.

11. Procédé selon l'une des revendications 7 à 10, dans lequel la température est comprise entre 10 et 80°C.

12. Procédé de préparation des composés selon la revendication 2, qui consiste à faire réagir le composé de formule : 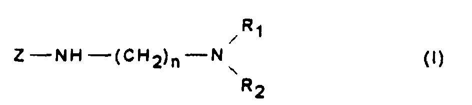
- avec un agent acylant de formule :
R₄―CO―R₅ (V)
dans lesquelles :
Z, R₁, R₂, R₄ et n ont la signification donnée précédemment et,
R₅ représente un atome d'halogène, un groupe O-CO-R₆ dans lequel R₆ est une chaîne préférence C₆-C₁₇, ou un groupe O-R₇ dans lequel R₇ est un radical alkyle en C₁-C₄ ou diglycéryle dont les résidus acyles, identiques ou différents, renferment 2 à 22 atomes de carbone.

13. Procédé selon la revendication 12, dans lequel R₆ est une chaîne hydrocarbonée en C₆-C₁₇ et R₇ est un résidu glycéryle dont les résidus acyles renferment 7 à 18 atomes de carbone.

14. Procédé selon la revendication 12, dans lequel le rapport molaire du composé de formule (I) à l'agent acylant est compris entre 1:0,95 et 1:4.

15. Procédé selon la revendication 12, dans lequel l'agent acylant est un chlorure d'acide ou un anhydride d'acide, et la réaction est effectuée à la pression atmosphérique à une température comprise entre -10 et +60°C.

16. Procédé selon la revendication 12, dans lequel l'agent acylant est un ester, et la réaction est effectuée à une pression de 0,013 à 101,325 kPa et à une température comprise entre 60 et 150°C.

17. Procédé selon la revendication 12, caractérisé en ce que la réaction est effectuée à un pH compris entre 7 et 11.

18. Procédé de préparation des composés selon la revendication 3, qui consiste à faire réagir le composé de formule Z-OH,
- avec une amine de formule : 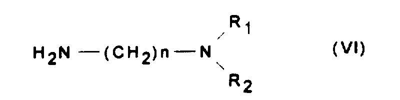 dans lesquelles Z, R₁, R₂ et n ont la signification donnée précédemment, le rapport molaire du saccharide à l'amine étant compris entre 1:0,90 et 1:1,5, de préférence 1:0,95 et 1:1,2

19. Procédé selon la revendication 18, caractérisé en ce que la réaction est effectuée dans un solvant choisi parmi l'eau, un alcool à chaîne courte ou un mélange hydroalcoolique.

20. Procédé selon la revendication 18 ou 19, dans lequel on utilise un monosaccharide en solution dans un alcool ou un disaccharide en solution dans un mélange hydroalcoolique.

21. Procédé selon la revendication 18, dans lequel la température est comprise entre 10 et 80°C.

22. Composition cosmétique ou capillaire comprenant un ou plusieurs composés selon les revendications 1 et/ou 3, un véhicule et, le cas échéant, des additifs.

23. Composition selon la revendication 22, dans laquelle le ou les composés représentent 0,1 à 50 % en poids de la composition.

24. Utilisation des composés selon l'une des revendications 1 ou 3 comme agents de conditionnement du cheveu.

25. Utilisation des composés selon l'une des revendications 1 ou 3 comme adjuvants de mouillage ou de broyage.

26. Utilisation des composés selon l'une des revendications 1 ou 3 comme agents de floculation ou de précipitation.

27. Utilisation des composés selon l'une des revendications 1 ou 3 comme dispersants de pigments.
